# EUROPEAN PATENT SPECIFICATION

(11) **EP 2 341 896 B1**
(45) Date of publication and mention of the grant of the patent: **09.09.2020**
(21) Application number: 09817269.5
(22) Date of filing: 01.10.2009
(51) Int. Cl.: A61K 9/16, A23P 10/25

(54) **LIPID PELLETS WITH ENHANCED TASTE-MASKING**
LIPID-PELLETS MIT VERBESSERTER GESCHMACKSMASKIERUNG
GRANULÉS LIPIDES AVEC MASQUAGE DE GOÛT AMÉLIORÉ

(30) Priority: 01.10.2008 EP 08017286; 01.10.2008 US 101717 P
(43) Date of publication of application: 13.07.2011
(73) Proprietor: Breitkreutz, Jörg, 45721 Haltern am See (DE); Matilainen, Julia, 79639 Grenzach-Wyhlen (DE)
(72) Inventor: Breitkreutz, Jörg, 45721 Haltern am See (DE); Matilainen, Julia, 79639 Grenzach-Wyhlen (DE)
(74) Representative: Kröncke, Rolf
(86) International application number: PCT/EP2009/007047
(87) International publication number: WO 2010/037543

(56) References cited:
- EP-A- 1 970 066
- WO-A-2008/148484
- KRAUSE JULIA ET AL: "Immediate release pellets with lipid binders obtained by solvent-free cold extrusion" EUROPEAN JOURNAL OF PHARMACEUTICS AND BIOPHARMACEUTICS, vol. 71, no. 1, Sp. Iss. SI, 5 September 2008 (2008-09-05), pages 138-144, XP002517498 ISSN: 0939-6411 cited in the application
- REITZ C ET AL: "Spheronization of solid lipid extrudates" POWDER TECHNOLOGY, ELSEVIER SEQUOIA, LAUSANNE, CH, vol. 189, no. 2, 16 April 2008 (2008-04-16), pages 238-244, XP025884753 ISSN: 0032-5910 [retrieved on 2008-04-16]
- REITZ C ET AL: "Solid lipid extrudates as sustained-release matrices: The effect of surface structure on drug release properties" EUROPEAN JOURNAL OF PHARMACEUTICAL SCIENCES, ELSEVIER, AMSTERDAM, NL, vol. 35, no. 4, 14 August 2008 (2008-08-14), pages 335-343, XP025560081 ISSN: 0928-0987 [retrieved on 2008-08-14]
- BREITKREUTZ J ET AL: "Pediatric drug formulations of sodium benzoate: - II. Coated granules with a lipophilic binder" 1 September 2003 (2003-09-01), EUROPEAN JOURNAL OF PHARMACEUTICS AND BIOPHARMACEUTICS, ELSEVIER SCIENCE PUBLISHERS B.V., AMSTERDAM, NL, PAGE(S) 255 - 260 , XP004453360 ISSN: 0939-6411 cited in the application the whole document

## Description

The present invention relates to pellets obtainable by a method for preparing solid pellets by cold solvent-free extrusion. Said pellets are characterized by having spherical shape and a lipid coating formed completely on the surface. Moreover, said pellets provide enhanced taste-masking of e.g. acitive ingredients present in said pellets. In another aspect, methods for obtaining said pellets are provided. In addition, the present application relates to lipid binder compositions for pellets allowing the preparation of release-controlled pellets having enhanced taste-masking properties.

### Background of the invention

The taste of a formulation is an important aspect in drug development. Taste has got a major influence on patient compliance, especially in the paediatric population. Taste masking can be defined as a perceived reduction of an undesirable taste that would otherwise exist (1). Various techniques can be used to achieve this reduction with differing success. There are numerous papers and patents in the literature on taste masking of pharmaceuticals.

A feasible and widely used option for the taste masking of solid formulations is the coating with polymers to achieve a physical barrier and thus minimizing the contact between drug and taste buds. One drawback is the additional process step, as well as the use of excipients with sometimes low acceptable daily intake values (ADI) or critical toxicological evaluation, especially in case of the chronic use of paediatric medication, and the need for solvents in this process step. Various coating agents such as cellulose derivates (HPC, HPMC, CMC, EC), methacrylic acid copolymers (Eudragit) or shellac are used for taste suppression of bitter tasting drug and are described in the literature (1).

A similar approach is the taste masking through a lipid coating, thus using excipients with low concerns regarding toxicity. Faham et al. (2) coated granules with molten glycerol dibehenate and Sugao et al. (3) coated microparticles with a hydrogenated oil and surfactant mixture in a fluidized bed. Incorporation of drug in lipophilic matrices can also lead to improvement of taste, as Suzuki et al. (4) showed by preparing chewable tablets with acetaminophen and hard fat (Witocan®) as the matrix base, resulting in suppressed bitterness of the tablets. The unpleasant taste of clarithromycin could be masked through incorporation into a wax matrix consisting of 60 % glycerol monostearate and 10 % aminoalkyl methacrylate copolymer A (5).

Recently Breitkreutz et al. (6) described a granule formulation useful as pediatric drug formulation of sodium benzoate containing sodium benzoate as active ingredient and 20 % hard fat additionally coated with a saliva-resistant coating to mask unpleasant taste of the sodium benzoate. Lipids as excipients are described in the art. Moreover, Krause et. al., (8) mentioned immediate release pellets, meaning granules with spherical shape, with various combinations of lipid binder compositions obtained by solvent-free cold extrusion. The pellets described therein release the active ingredient immediately at 37°C. However, Krause et. al., are silent about the coating of the pellets and the problem of taste masking.

As outlined before, there is an ongoing need for coating of pellets for taste masking. The aim of the present invention is to provide an economical process allowing the production of taste-masked formulations while reducing costs and time of preparing the same. In another aspect, the present invention aims to provide binder compositions and pellets containing said binder compositions allowing a controlled release of the active ingredient in said pellets. These and other problems are achieved by the underlying invention.

### Brief description of the present invention

In a first aspect, the present invention relates to pellets or spherical granules having a coating of a lipid binder composition obtainable by cold solvent-free extrusion comprising the steps of
a) mixing the active ingredient with said lipid binder composition comprising at least (i) a hard fat, (ii) either glycerol trimyristate or glycerol distearate, and, (iii) optionally, glycerol dibehenate,
b) cold extruding the mixture,
c) spheronising the extrudate to obtain spherical pellets or granules, characterized in that the coating is formed on the surface of said pellets or granules covering completely said pellets or granules during spheronisation of the extrudate and that the coating does not substantially contain any active ingredients which are released at 24°C within 20 min.

In a further aspect, the present invention relates to methods for the preparation of said pellets or granules according to the present invention. Finally, the present invention relates to lipid binder compositions for pellets or granules obtainable by cold solvent-free extrusion according to the present invention comprising at least (i) a hard fat, (ii) either glycerol trimyristate or glycerol distearate and, (iii) optionally, glycerol dibehenate.

### Brief description of the drawings

Figure 1: Figure 1a: Influence of dissolution media temperature on release characteristics of lipid sodium benzoate pellets. Formulation W20 (mean i SD, n=6), Dissolution media: purified water, 150 rpm, Basket method. Figure 1b: Influence of dissolution media temperature on release characteristics of lipid sodium benzoate pellets. Formulation W15 P2.5 D2.5 (mean 1 SD, n=6), Dissolution media: purified water, 150 rpm, Basket method.
Figure 2: Figure 2a: Influence of dissolution media temperature on release characteristics of lipid sodium benzoate pellets. Formulation W15 P2.5 D2.5 (mean i SD, n=6), Dissolution media: purified water, 150 rpm, Basket method. Figure 2b: Influence of dissolution media temperature on release characteristics. Dissolution media: purified water (mean + SD, n=6), 150 rpm, Basket method. Figure 2c: Influence of dissolution media temperature on release characteristics. Dissolution media: purified water (mean 1 SD, n=6), 150 rpm, Basket method.
Figure 3: Influence of dissolution media temperature on release characteristics of lipid pellets. Dissolution media: 0.1 N HCI-solution pH 1 (mean + SD, n=6), 150 rpm, Basket method.
Figure 4: Scanning electron micrographs of pellet formulation W15 D5. (A) Pellet after extrusion/spheronisation (B) pellet after dissolution at 24 °C, (C) Pellet after dissolution at 37 °C.
Figure 5: DSC tracks of different lipid pellets, recorded at a heating rate of 10 K/min. All signals are scaled to sample weight.
Figure 6: Scanning electron micrographs of a pellet containing _80% sodium benzoate, 15% Witocan and 5% Compritol (formulation W15 C5).
Figure 7: Dissolution profile of pellets containing 80 % sodium benzoate, 15 % Witocan, 2.5 O/O Precirol and 2.5 % Dynasan (W15 P2.5 D2.5). Milled pellets: Percentage of actual released sodium benzoate; Intact pellets: Percentage of labelled sodium benzoate content. Dissolution media: purified water (mean i SD, n=3), Temperature 24 °C, 150 rpm, Basket method.
Figure 8: Release of sodium benzoate from two different pellet formulations, 2g pellets mixed with 100g food stuff (n = 3, mean i SD).

### Detailed description of the present invention

In a first aspect, the present invention relates to spherical pellets or spherical granules having a coating of a lipid binder composition obtainable by cold solvent- free extrusion and subsequent spheronisation comprising the steps of.
a) mixing the active ingredient with said lipid binder composition comprising at least (i) a hard fat, (ii) either glycerol trimyristate or glycerol distearate, and, (iii) optionally, glycerol dibehenate,
b) cold extruding the mixture,
c) spheronising the extrudate to obtain pellets or spherical granules, characterized in that the coating is formed on the surface of said pellets or granules covering completely said pellets or granules during spheronisation of the extrudate and that the coating does not substantially contain any active ingredients which are released at 24°C within 20 min.

That is, the present inventors recognized that by using the specific lipid binding composition according to the present invention for cold extrusion and, thereafter, spheronising the extrudate to obtain spherical pellets or granules, a lipid coating is formed on the surface of said pellets or granules covering totally or completely, respectively, the pellets or granules, while the starting material, namely the mixture of active ingredient and the lipid binder composition, are homogenously mixed. Said lipid coating is reduced in its content of active ingredient compared to the content of the active ingredient in the pellets cores, preferably, the coating is substantially absent of active ingredients which are released at 24°C within 20 min according to the test in purified water at 150 rpm using the dissolution test apparatus Ph. Eur./USP equipped with the Basket method.

The terms "substantially absent" or "does not substantially contain" as used herein refer to the aspect that the pellets or granules according to the present invention do release the active ingredient at a temperature of 24 °C in an amount of 1 % at most, preferably 0.5% at most, like 0.2% at most after 20 minutes determined in purified water at 150 rpm using the dissolution test apparatus Ph. Eur./USP equipped with the Basket method.

In this connection, the term "pellet" and "granule" are used to describe a variety of agglomerates. Pellets in the pharmaceutical context are defined as isometric agglomerates with a smooth surface and a narrow size distribution. Typically, the pellets or granules are in the range of from 10 pm to 1500 pm, like 200 or 1000 pm. Granules are solid agglomerates, made from powders with or without liquid binders. Pellets are isometric granules. Unless otherwise indicated, the term pellet refers to pellets as well as granules.

There are several preparation methods for pellets or granules known in the art. Two main processes exist; the first one is a direct pelletisation, either wet pelletisation using a liquid binder or melt pelletisation using higher temperatures to melt the binding agent. This is usually performed in high-shear mixer/granulators or in fluidized bed granulators. Another method is the layering of seeds which leads to heterogenous pellets or granules with a core and a shell.

Extrusion is meant to describe a process were plasticized material is pressed through defined openings. Until recently, extrusion can be differentiating into two main process types, wet and melt extrusion as indicated above. Pellets or granules according to the present invention are produced by an extrusion/spheronisation process recently described by the present inventors (6) which is incorporated herein by reference.

Solvent-free extrusion in the present application means that no wetting of the mixture of active ingredient and lipid binder composition takes place and, consequently, no drying of the pellets after extrusion is required.

Cold extrusion means that the extrusion temperature is selected in a way that the temperature is below the melting point of the lipids contained in the binder solution according to the present invention.

As noted above, the pellets prepared according to the method of the present invention are characterized in that a lipid coating is formed on the surface of said pellets enveloping or covering the pellets fully or completely, respectively. Said coating is formed during the speronisation process.

Said lipid coating has preferably a thickness of at least 5pm, like at least 7pm, for example at least 10 pm. The coat is characterized in that the ratio of active ingredient to lipid binding composition is drastically reduced compared to said ratio in the pellet itself, preferably, the active ingredients are substantially absent in the coating. That is, the coating may contain 5 wt-%, like 2 wt-%, e.g. 1 wt-% or 0.5 wt-% or less of the active ingredient. Thus, taste-masking of the active ingredients present in the pellets is achieved.

The spheronisation step according to the present invention is conducted using typical spheroniser. During the spheronisation process spherical pellets are obtained. The spheronisation parameters to obtain spherical pellets comprises the spheroniser temperature including the temperature of the jacket and the plate, the speed and the time interval of spheronisation.

Preferably, the spherical pellets obtained according to the present invention have an aspect ratio (AR) of lower or equal to 1.2, like equal or lower 1.1. In this connection, the aspect ratio refers to the length with ratio of the pellets. That is, the aspect ratio is calculated from AR=dmax/d90 with maximum Feret diameter (dmax) and the Feret diameter perpendicular to it (d90).

In a preferred embodiment, the method for preparing the pellets or granules does not include any processing step for further coating of the pellet after spheronisation. That is, the present inventors developed a one-step process for preparing pellets or spherical granules according to the present invention by cold solvent-free extrusion for providing taste-masked pharmaceutical formulations.

In a preferred embodiment, the lipid binder composition used for admixture with the active ingredient comprises at least a hard fat and glycerol distearate.

The term "hard fat" as used herein refers to a hard fat as defined in the European Pharmacopoeia in its present edition (the update version is 6.2). A typical example of a hard fat useful according to the present invention is the product "Witocan® 42/44 microfein", Sasol, Deutschland. Other examples useful as hard fats according to the present invention comprise "Witepsol", Sasol, Germany. A hard fat preferably has a melting point of from 40 to 44°C. Hard fats are characterized in being triglycerides from animal or plant origin and characterized by different characteristic values in the pharmacopoeias.

The term "glycerol distearate" comprises pure as well as industrially produced glycerol distearate including mixture of mono, di-, and tri-distearate. The term is defined according to Ph. Eur. A typical example of glycerol distearate used according to the present invention is glycerol distearate (Precirol® ATO5), Gattefosse GmbH, Weil am Rhein, Deutschland.

The term "glycerol trimyristate" as used herein concerns composition containing mainly glyceryl trimyristate, for example, containing more than 95 % triglyceride. The term is defined according to Ph. Eur. A typical example of a glycerol trimyristate used according to the present invention is Dynasan® 114 from Sasol, Deutschland. The glycerol trimyristate has typically a melting point of about 55 to 58 °C.

Further, the term "glycerol dibehenate" refers to glycerol dibehenate compositions containing mainly glycerol dibehenate optionally in combination with glycerol tribehenate and triglycerolmonobehenate. The term is defined according to Ph. Eur. Preferably, the glycerol dibehenate has a melting point of about 69 to 74 °C. A typical example of a glycerol dibehenate used according to the present invention is Compritol® 888 ATO of Gattefosse, Weil am Rhein, Deutschland.

In a preferred embodiment, the lipid binder composition comprises
to 90 weight % hard fat
0 to 30 weight % glycerol trimyristate
0 to 30 weight % glycerol distearate
0 to 30 weight % glycerol dibehenate
whereby either glycerol trimyristate or glycerol dibehenate is present in an amount of at least 0.1 wt-%.

In another preferred embodiment, the amount of active ingredient in the pellet is in the range of from 0.1 to 90 wt.-%, like 10 to 80 weight-% of the total pellet.

Preferably, the active ingredient is characterized in being a hydrophilic active ingredient or pharmaceutically acceptable salt thereof. That is, the active ingredient is in a hydrophilic form either as the base or in form of a pharmaceutically acceptable salt thereof, allowing dissolution in aqueous liquids. Typically, the active ingredient or its pharmaceutical acceptable salt from the biopharmaceutical classification system (BCS), class 1 and class 3.

In another preferred embodiment the spheronisation of the extrudate in the spheroniser is performed for at least 15 minutes, like at least 20 minutes.

In a further preferred embodiment, the temperature of the jacket of the spheroniser is at least 5 °C below the melting temperature of the lipid component of the lipid binding composition having the lowest melting temperature. Preferably, the temperature is in between 15 to 5 °C, like in between 10 °C and 5 °C below the lowest melting temperature of any of the components of the lipid binding composition.

That is, in case of using the hard fat component Witocan® having a melting temperature of 42 to 44 °C, the temperature of the jacket of the spheroniser is in a range of from 30 °C to 37 °C , like 32 °C to 37 °C. In case, the range between the temperature of the jacket and the melting temperature is less than 5 °C, sticking of the extrudate to the spheroniser resulting in a single pellet may occur. On the other hand, in case the temperature is more than 10 °C below the lowest melting temperature, the formation of the lipid layer on the surface of the pellet may not occur

During spheronisation, the spheronsiation plates speeds preferably are between 750 rpm and 1500 rpm.

Extrusion and spheronisation may be conducted with any device known in the art suitable for extrusion and spheronisation of pharmaceutical pellets.

In another aspect, the present invention relates to pellets obtainable by a process according to the present invention whereby these pellets comprising the active ingredient and the lipid binder composition comprising at least a hard fat, either glycerol trimyristate or glycerol distearate, and, optionally, glycerol dibehenate, are characterized in displaying excellent taste-masking and allowing regulation of drug release. In particular, the present inventor recognized that the composition of the lipid binder allows to regulate and control drug release, in particular, allow to provide pellets and granules of delayed type release or pellets and granules showing different release characteristics at different temperatures as described herein.

Moreover, the pellets or granules according to the present invention have improved shelf-life. That is, the active ingredients present in the core are protected against hydrolysis and other types of deterioration of said ingredients. Further, the lipid coating provides protection of the oral mucosa against the active ingredients present in the pellets or granules which is desired in case of e.g. oral mucosa irritant compounds, like very acid or very basic agents or in case of immuno-suppressive or cytotoxic agents. Thus, the pellets or granules according to the present invention having a coating completely covering the cores of the pellet or granules allows to taste-masking the active ingredients as well as avoiding undisered side-effects of known pellets or granules for oral administration. The pellets or granules are particularly suitable for oral administration.

In a preferred embodiment, the pellet according to the present invention are characterized in having a lipid layer formed on surface of the pellet which layer has a thickness of at least 5 pm, like at least 7 pm, for example at least 10 pm.

Preferably, the active ingredient present in the pellet is an active ingredient of hydrophilic nature. The active ingredient may be present in its base form or as a pharmaceutical acceptable salt.

In a particular preferred embodiment the pellet is characterized in that the drug release at a temperature of 24 °C is 1 °/0 at most after 20 minutes dissolution in purified water at 150 rpm according to the basket test as identified in the Ph. Eur. Moreover, it is preferred that the drug release at a temperature of 37°C is above 90 wt-% after 45 min determined in purified water at 150 rpm using the dissolution test apparatus Ph. Eur./USP equipped with the Basket method.

The pellets according to the present invention are characterized in that the concentration or content of active ingredient in the lipid layer encapsulating the pellet is lower than the concentration of the active ingredient in the pellet as identified above.

Furthermore, the pellet according to the present invention are characterized in that substantially no or only a little release of the drug can be observed at a lower temperature while at higher temperature immediate or sustained release occurs. For example, as shown herein, the drug release at a temperature of 24 °C, i.e. at room temperature, is substantially deferred while at a higher temperature, here 37 °C, i.e. body temperature, release of the drug occurs within 20 to 60 minutes. Further, the composition of the lipid binder composition allows regulating the release of the active ingredient. For example, compositions containing the hard fat in combination with the glycerol trimyristate and the glycerol dibehenate have a deferred release in comparison to a binding composition composed of hard fat and glycerol distearate or of hard fat and glycerol trimyristate only.

In a further aspect, the present invention relates to a method for the production of the pellets or granules according to the present invention as defined herein.

Finally, the present invention relates to the lipid binder composition for pellets itself comprising at least a hard fat, either glycerol trimyristate or glycerol distearate, and, optionally, glycerol dibehenate. Preferably, the lipid binder composition comprises at least hard fat and glycerol distearate.

In a preferred embodiment of the lipid binder composition, the composition comprises
0 to 90 wt-% hard fat.
0 to 30 wt-% glycerol trimyristate
0 to 30 wt-% glycerol distearate
0 to 30 wt-% glycerol dibehenate
whereby either glycerol trimyristate or glycerol distearate are present in an amount of at least 0.1 wt-%.

In a particularly preferred embodiment, the lipid binder composition is composed of 75 wt-% hard fat, 12.5 wt-% glycerol trimyristate and 12.5 wt-% glycerol distearate.

Variations in the exact composition of the lipid binder composition allow regulating drug release, dissolution properties and formation of the lipid layer on the surface of the pellet. Further, the composition of the lipid binder composition depends on the active ingredient used in the pellet. That is, depending on the hydrophilic of the active ingredient, the lipid binder composition may be determinant accordingly.

The following examples serve to exemplify the invention but should not be constituted as a limitation therefore.

### Materials and Methods

### Materials

The following materials were used in Ph. Eur. Grades: Sodium benzoate (SB) from Ethicare GmbH (Haltern am See, Germany); powdered hard fat (Witocan® 42/44 mikrofein) and glyceryl trimyristate powder (Dynasan® 114) from Sasol GmbH (Witten, Germany); glyceryl palmitostearate powder (Precirol® ATO 5) and glyceryl dibehenate powder (Compritol® 888 ATO) from Gattefosse GmbH (Weil am Rhein, Germany); acetic acid from Carl Roth GmbH (Karlsruhe, Germany); acetonitrile from Fisher Scientific (Loughborough, UK) and ammonium acetate from Riedel-de-Haen (Seelze, Germany). Benzoic acid RS (USP 05500), lot. F-5, was used as the primary reference standard for all measurements of sodium benzoate contents and concentrations.

### Extrusion and spheronisation

After weighing, the dry powders were blended for 15 min in the laboratory scale blender LM 40 (Bohle, Enningerloh, Germany) and then transferred to the gravimetric powder feeder of the twin-screw extruder Mikro 27GL-28D (Leistritz, Nuremberg, Germany). The extruder was equipped with an axial screen with 23 dies of 1 mm and 2.5 mm length. The extrusion of lipid mixtures was performed at a constant screw speed of 50 rpm and a powder feed rate of 40 g/min. Batches of 300 g extrudate were collected and then spheronised at 1500 rpm for 15 min in the spheroniser RM 300 (Schltiter, Neustadt, Germany) fitted with a cross-hatched rotor plate of 300 mm diameter.

### Drug Release

The dissolution tests were performed using the Ph. Eur. basket apparatus method at 150 rpm (Sotax AT7, Sotax, Lorrach, Germany). Preliminary investigations revealed no significant influence of the rpm speed on the dissolution profile. The dissolution media were purified water, 0.1N HCI pH 1 and phosphate buffer solution pH 6.0 R2 (Ph. Eur) continuously pumped (Sotax piston pump CY 7, Sotax, Lorrach, Germany) to a UVNIS-Photometer (Lambda 2, Perkin-Elmer, Uberlingen, Germany). The phosphate buffer pH 6.0 was selected from chapter 2.9.25 Eur. Ph. "Dissolution test for medicated chewing gums" to simulate drug release in the oral cavity. The concentration of sodium benzoate in the dissolution fluid was determined by measuring the absorption at 273 nm. Preliminary investigations showed that the extrusion aids do not have an impact on the absorption at the wavelength 273 nm. All experiments were conducted with five replicates.

### Scanning electron microscopy

Pellets were visualised by the scanning electron microscope Leo 1430 VP (Leo Electron Microscopy, Cambridge, UK). The samples were gold sputtered by the Agar Manual Sputter Coater B7340 (Agar Scientific, Stansted, UK) prior to electron microscopic investigations.

### HPLC

Sodium benzoate content in the food samples was determined by a validated high-performance liquid chromatography (HPLC) method, described by Breitkreutz (7). 2 g pellets from each batch, equivalent to 1.6 g sodium benzoate, which is a typical single dose for a one-year-old infant, were mixed with 100 g food. Samples of the food were taken after 5, 10 and 20 minutes in between intermittent stirring. A sample of 1g, accurately weight, was dissolved in 100 ml distilled water in a volumetric flask, undissolved food content was sieved off; 5.0 ml of the solution was diluted to 100 ml with the HPLC eluent, a mixture of 30% acetonitrile and 70% 0.01 M aqueous ammonium acetate solution adjusted to pH 4.5 by acetic acid. 20 microliters of the solution were injected into the HPLC apparatus Hewlett-Packard 1090 Series II (Agilent, Boblingen, Germany) adjusted to 40 °C, equipped with a reverse phase prepacked column Agilent Eclipse XDB-C18 (dimensions: 4.6 x 150 mm, size of packaging material: 5 pm, Agilent) and an UV-diode array detector. Flow rate was 1.0 ml/min. The sodium benzoate content was calculated by the area under the curve of clearly resoluted absorption peaks at a retention time from 3.7 to 3.8 min compared to external calibration standards of the pure drug treated in the same manner.

### Differential scanning calorimetry (DSC)

Thermal characteristics of the powdered lipids were studied using a Mettler DSC 821e (Mettler Toledo, Giessen, Germany). DSC scans were recorded at a heating rate of 10 °C/min. Samples with an initial weight of approximately 3 mg were heated from 20 to 120 °C in a sealed and pierced aluminium pan. An empty aluminium pan was used as reference. Dissolution tests with prior art formulations of 80 % sodium benzoate and 20 % hard fat revealed immediate release profiles which could be explained by the high drug load and the good water solubility of sodium benzoate (6). The prior art formulations needed a saliva-resistant coating to mask the unpleasant bitter and salty taste. The newly described formulations provide pellet formulations which, when stirred in water at room temperature, displayed no taste. To analyse these findings dissolution tests at standard dissolution media temperature of 37 °C and also at 24°C, simulating room temperature, were performed for all seven batches as shown in table 1.

**Table 1**

| Formulation | Sodium benzoate [%] | Witocan 42/44 [%] | Compritol 888 ATO [%] | Dynasan 114 [%] | Precirol ATO 5 [%] |
|---|---|---|---|---|---|
| W 20 | 80 | 20 | | | |
| W 15 C 5 | 80 | 15 | 5 | | |
| W 15 D 5 | 80 | 15 | | 5 | |
| W 15 P 5 | 80 | 15 | | | 5 |
| W 15 C 2.5 D 2.5 | 80 | 15 | 2.5 | 2.5 | |
| W 15 P 2.5 C 2.5 | 80 | 15 | 2.5 | | 2.5 |
| W 15 P 2.5 D 2.5 | 80 | 15 | | 2.5 | 2.5 |

| | | | | | |
|---|---|---|---|---|---|
| *W* Witocan 42/44 P Precirol ATO 5 D Dynasan 114 C Compritol 888 ATO Numbers indicate the percental fraction of the lipid in the formulation | | | | | |

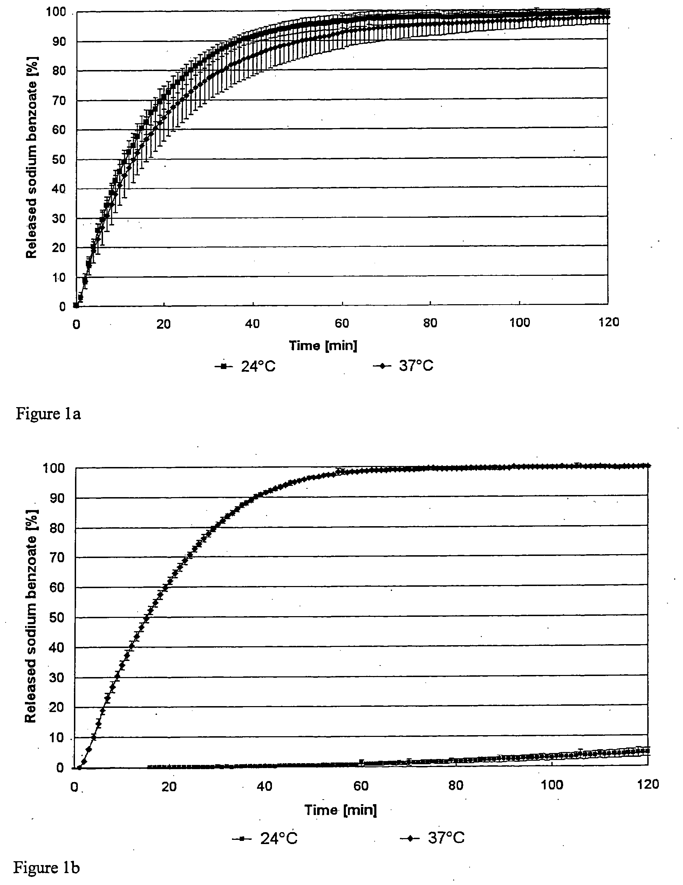

As expected, the dissolution tests performed with pellets consisting of SB and hard fat revealed no influence of the dissolution media temperature, as can be seen 25 in Figure 1a.

At both temperatures more than 40 °/0 of the drug were released within the first 40 min. Further tests were performed with a pellet batch containing the lipid mixture of hard fat, glycerol distearate and glycerol trimyristate, this combination led to optimal pellet characteristics concerning spherical shape and narrow particle size distribution. As can be seen in Figure 1b, a temperature of 37 °C led to complete drug release within 60 min, as could be seen with the reference pellet batch, but a lower temperature of 24 °C led to a drug release of less than 5 % within the tested time period of two hours. The observed absence of a taste at room temperature could therefore be explained by the non-existing release from these pellets at room temperature.

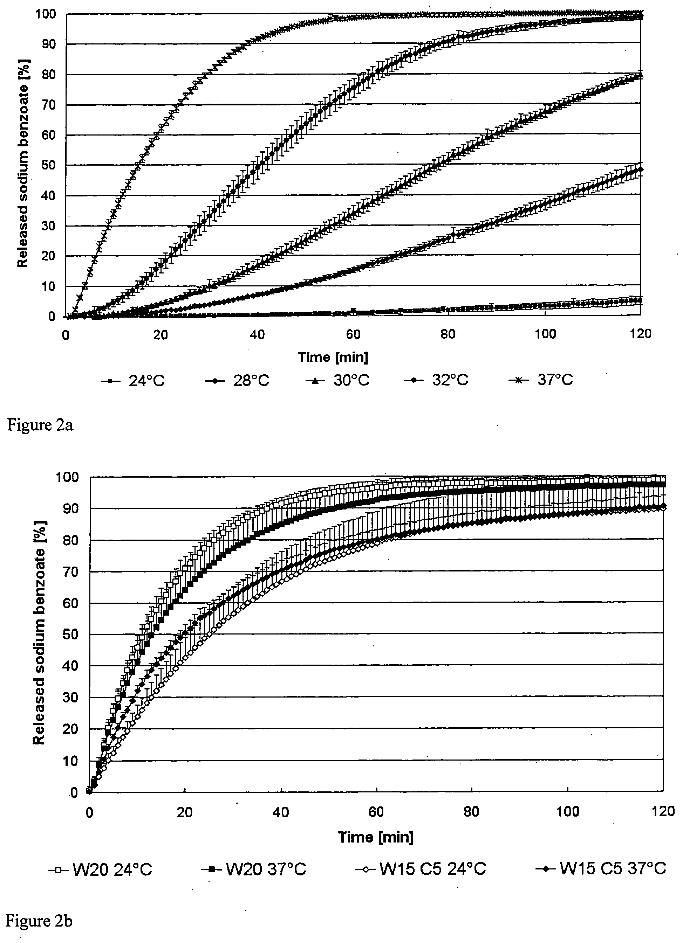

To obtain further insight into the mechanism behind this phenomenon dissolution profiles between the basic data at 24 °C and 37 °C were investigated. The dissolution media was set to three intermediate temperatures of 28, 30 and 32 °C, as can be seen in Figure 2a. With an increase in temperature of the dissolution media, the drug release increases. There is no threshold temperature to reach maximum release, rather a gradual increase. Consequently all other lipid batches were also tested under these conditions and formulations could be divided in two groups. The pellet batches containing hard fat or a mixture of hard fat and glycerol dibehenate displayed no temperature sensitive release profiles, displayed in Figure 2b. The release profiles remained similar and did not vary by temperature change for these two batches. Again more than 80 % of the drug content was released within the first 60 min.

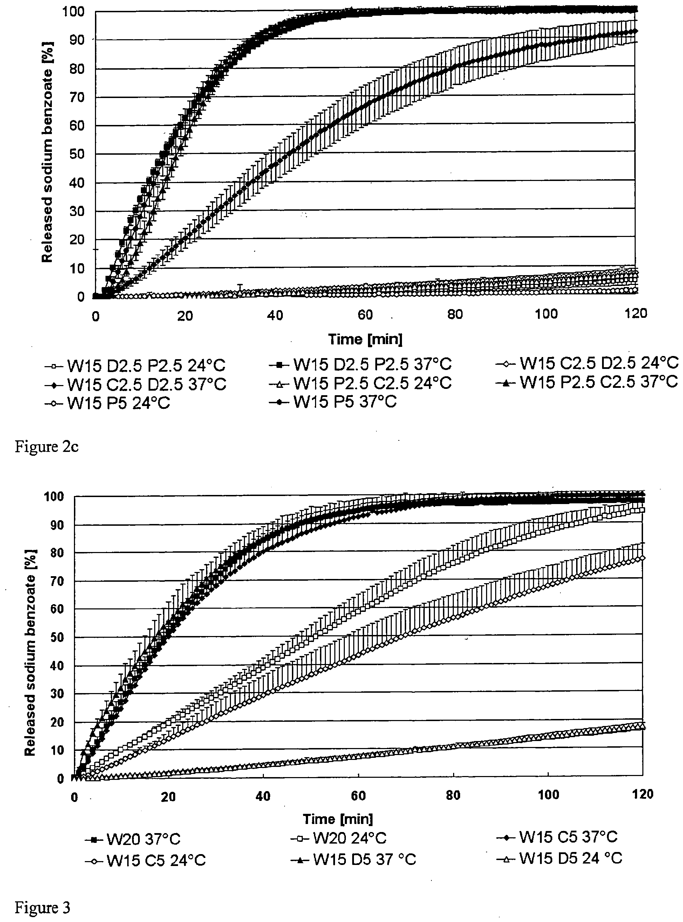

In contrast, as can be seen in Figure 2c, the other two binary and three ternary mixtures exhibited a significant influence of media temperature on the dissolution characteristics. Less than 10 % drug release over two hours could be observed in any of these pellet batches for a dissolution media temperature of 24 °C.

Furthermore, the influence of the dissolution media type on the drug release from lipid pellets was investigated. The release under room temperature conditions varied with the media type, depending on the lipid mixture. Three batches, two binary mixtures with hard fat and either glycerol trimyristate or glycerol dibehenate and the reference batch with 20 % hard fat, showed a delayed drug release in the acidic media of 0.1 N HCI, as can be seen in Figure 3. Sodium benzoate can form slightly soluble_benzoic acid in acid environment. If benzoic acid is formed during dissolution, the pellet surfaces will be coated by the crystals and the release might be delayed through this effect. For all three ternary mixtures as well as the binary mixture of hard fat and glycerol distearate this effect was not observed. No significant changes in the release profiles occured when the media type was changed to phosphate buffer with a pH of 6 (data not shown).

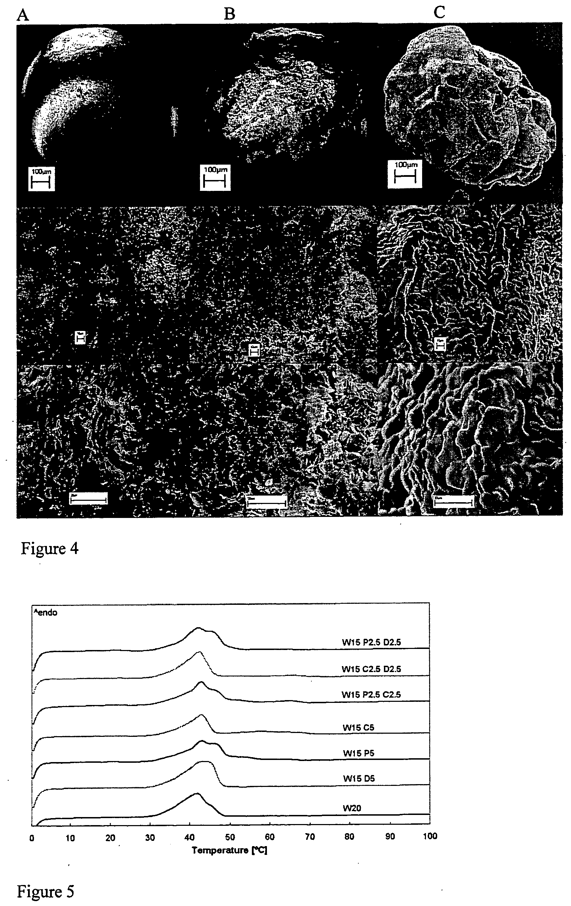

To further investigate the discovered effects, pellets were observed by scanning electron microscopy. The micrographs of lipid pellets display several different surface features independently from the used lipid mixture. The lipid pellets have mostly a smooth surface, indicating molten lipids, which is repeatedly broken by pore-like structures. All pellet batches displayed these features irrespective of their temperature dependent dissolution. To further analyse differences in surface structure the pellets were also investigated after dissolution. Scanning electron microscope analysis of lipid pellets before and after dissolution at different temperatures revealed interesting differences in the surface appearances. In Figure 4 scanning electron micrographs of untreated pellets, pellets after dissolution at 24 °C and pellets after dissolution at 37 °C from the batch W15 D5 can be compared.

Pellets released at a temperature of 24 °C show a similar surface structure as untreated pellets of the same batch. In smaller magnifications (40x; as adjusted at the microscope) no differences are apparent. Larger magnifications (400x, 1000x) show differences, like a flaked surface with more pores and needle-like structures, which are probably recrystallised sodium benzoate. In clear contrast to this are the surfaces of pellets after release in 37 °C media. Here even at small magnifications the pellets look shrunk and deformed. At a closer look none of the previous described features are present. The surfaces have a molten-like structure with no visible pores. As 37 °C is close to the melting point of the main binder hard fat, it is possible that the lipids start melting during dissolution forming such surfaces. Again, independently from the composition of the lipid mixture, the pellet surfaces have the same characteristics and features. An implication for the dissolution profile cannot be derived from the surface appearance.

To understand the mechanism behind the observed dissolution effects, especially the difference in the dissolution profiles at different temperatures, correlation with other pellet properties were investigated. The different dissolution profiles could not be correlated with the aspect ratio, a length-width ratio to describe the spherical shape of the respective pellet batches. The same applied to the porosity of the pellets, which was approximately 4 %. Another potential influence, the melting point of the used lipids, did also not determine the dissolution profile, e.g. the exchange of 5 % hard fat (melting point of 42-44 °C) with glycerol dibehenate, the lipid with the highest melting point of 73 °C, resulted in approximately the same dis- solution profiles which were not affected by temperature (see Figure 2b).

It is noticeable that glycerol distearate has a significant impact on the dissolution profile, as even small amounts (2.5 %) of this lipid in a formulation alters the dissolution at room temperature. This becomes also apparent in DSC measurements, as all pellet batches containing Precirol, irrespective of the respective amount or lipid mixture, display the same peaks or shoulders in the DSC measurements, as can be seen in Figure 5.

It is probable that the higher melting lipid binders dissolve in the molten or melting hard fat on the pellet surface at temperatures around 40 °C. During spheronisation this may lead to a plasticization of all lipids in the outer parts of the pellets, regardless their melting points and initial composition.

A possible mechanism to explain the observed effects is the presence of a lipid coating of the pellets. During the extrusion process, although no heat is used to plasticize the mixtures before the die passage, some parts of the lipid mixture melt due to the friction and pressure forces, especially on the extrudate surfaces. This effect is amplified in the spheronisation process where external heat and frictional heat of the pellet bed lead to partial melting of the binder and formation of spherical pellets.

A separation process between molten binder and the active crystalline drug takes places. The molten parts of the lipids layer in higher concentrations on the surfaces of the pellets, which leads to a coat of lipids around each pellet. Said coat is reduced in the amount of active ingredient. The composition of this coat, i.e. the amount of higher melting lipids dissolved in hard fat, determines the release behaviour of the respective pellet batch. The layer beneath this coat is slightly depleted of lipids whereas the pellet cores have an even distribution of binder and active drug. This mechanism of self-coating through the lipid binder is supported by pictures obtained through scanning electron microscopy of split pellets. It has not been reported in the literature up to now. The term self-coating has been previously used by the working group of Ulrich to describe an effect during hot melt technology where higher concentrations of the active are found on the sides than in the core of pastilles (6).

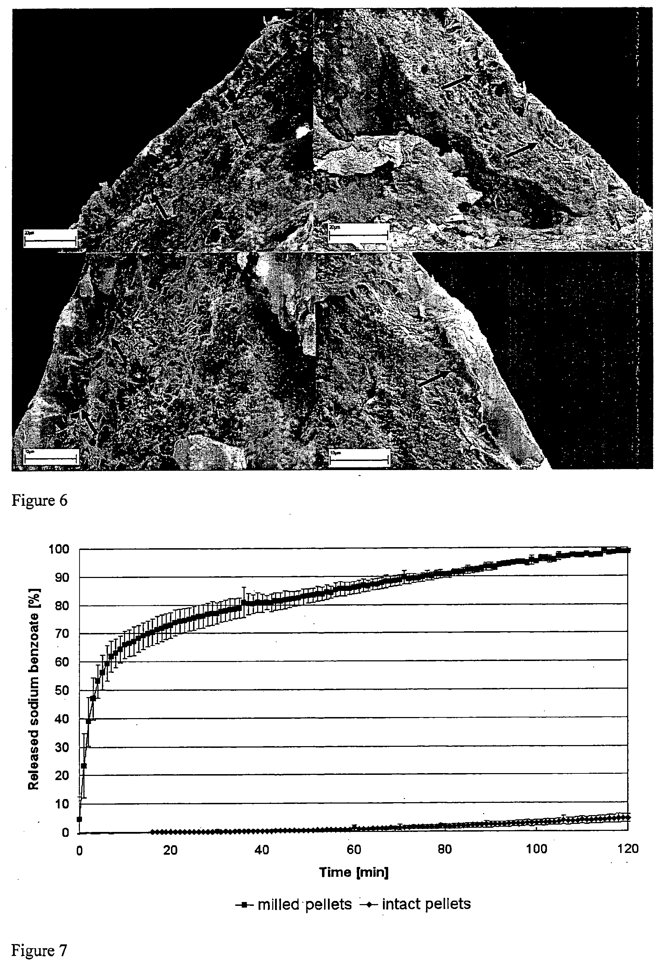

In Figure 6 the cross-section view of pellet halves is displayed. The black arrows point to the visible layer at the pellet edges. In all micrographs the same structures can be identified as an approximately 10 pm layer on the outer surface which is clearly visible. The outer layer appears congealed and smooth, whereas the second layer has a rather rough and even needle-like structure. This supports the hypothesis that a layer of drug-free or nearly drug free binder forms the outer layer followed by a layer with depleted binder content and therefore increased drug concentration.

Another verification of the hypothesis would be an increased drug release of milled pellets at room temperature of a batch, which showed no release at this temperature in intact conditions. The drug dissolution of pellets containing hard fat, glycerol distearate and glycerol trimyristate previously showing nearly no release at 24 °C over 2 hours, display a rapid release of sodium benzoate after milling i.e. destroying of the lipid coat (Figure 7). In this figure, unlike to before displayed release profiles, only the percentage of actual released sodium benzoate is shown, instead the percentage of labelled sodium benzoate content. Due to the milling a correct proportional initial weight of drug and binder could not be ensured and therefore no theoretical nominal content of sodium benzoate be calculated. The curves can still be compared as the kinetic of the release stays the same independent from the chosen data presentation. The temperature controlled release is therefore depending upon the presence of a lipid coat and controlled by it. The release can be modified by the composition of the used lipid mixture. Due to the temperature sensitive coating, it is possible to predetermine time and duration of the release of the active ingredients. Thus, the pellets and granules according to the present invention allow a temperature sensitive release of the active ingredients.

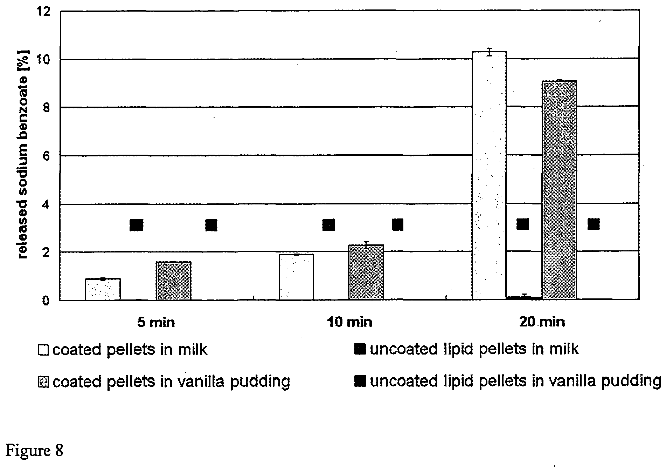

To further investigate the taste masking ability the release of sodium benzoate from the developed lipid pellets into different foodstuff was tested. The previously described pharmacopeial dissolution tests do not give evidence for the behaviour under in-vivo conditions. As a comparison the saliva-resistant coated granules, developed by Breitkreutz et al. (6), were used. A typical drug administration situation was simulated. 2 g pellets from each batch, equivalent to 1.6 g sodium benzoate, which is a typical single dose for a one-year-old infant, were mixed with 100 g food, either milk or vanilla pudding. Samples of the food were taken after 5, 10 and 20 minutes in between intermittent stirring. As can be seen from Figure 8, the release from the saliva-resistant coated pellets was higher than for the uncoated lipid pellets, where the release within the first 10 min was hardly detectable and even after 20 min still ranged under 1 %. The release for the coated pellet batch was about 1 % after 5 min, 2 % after 10 min and around 9-10 % after 20 min for the samples stirred in milk and vanilla pudding. The uncoated lipid pellets contained hard fat, glycerol distearate and glycerol trimyristate as binder (formulation W15 P2.5 D2.5) and were chosen due to their pronounced characteristics described before. The taste masking of lipid pellets can therefore be rated similar to pellets coated with amino-polymethacrylates (Eudragit E) and was even more efficient under typical drug administration conditions.

To conclude, the newly discovered principle of self-coating of lipid pellets, possible through the behaviour of the lipid binder during the extrusion/spheronisation process, offers interesting new aspects for the use in not only paediatric oral dosage forms. Taste masking through an additional salvia-resistant coating as described in the art can be avoided and the potential to design desired release profiles through the choice of lipid composition unseals new areas of application. E.G. it allows a variety of interesting possibilities to provide new modified release dosage forms without the need of an additional time- and resources-consuming coating step with potentially toxic excipients. Suitable fields of application are taste-masked suspensions, or granules and pellets for reconstitution of suspensions.

Through the variation of the lipid binders different release profiles can be obtained and through this effect desired drug release profiles can be designed. It is clear that especially glycerol distearate has an important influence on the described temperature dependent release. This temperature effect can result in taste masked drug formulations or other modified release dosage forms while still maintaining an immediate-release profile under physiological temperatures.

### References

[1] H. Sohi, Y. Sultana, K.R. Khar, Drug Dev. Ind. Pharm. 30 (2004) 429-448
[2] A. Faham, P. Prinderre, N. Farah, et al., Drug Dev. Ind. Pharm. 26 (2000) 167-176
[3] H. Sugao, S. Yamazaki, H. Shiozawa, et al., J. Pharm. Sci. 87 (1998) 96-100
[4] H. Suzuki, H. Onishi, S. Hisamatsu, et al., Int J Pharm 278 (2004) 51-61
[5] Yajima T, Nobuo U, Itai S, Chem Pharm Bull 47 (1999) 220-225
[6] J. Breitkreutz, M. Bornhoft, F. Woll, et al., Eur. J. Pharm. Biopharm. 56 (2003) 255-260
[7] E. Rombach, J. Ulrich, Reduktion der Prozessschritte durch unmittelbare Selbstbeschichtung von Partikeln, Chem-Ing-Tech 79 (2007) 215-222
[8] J. Krause, M. Thommes, J. Breitkreutz, Eur. J. Pharm. Biopharm. Doi: 10.1016/j.EJPB.2008.08.020 and 71: 138-144 (2009)

## Claims

1. The use of spherical pellets or spherical granules having a coating of a lipid binder composition for taste-masking obtainable by cold solvent-free extrusion comprising the steps of
a) mixing the active ingredient with said lipid binder composition comprising at least (i) a hard fat, (ii) either glycerol trimyristate or glycerol distearate, and, (iii) glycerol dibehenate,
b) cold extruding the mixture,
c) spheronising the extrudate to obtain pellets or spherical granules,
**characterized in that** the coating is formed on the surface of said pellets or granules covering completely said pellets or granules and that the coating does not substantially contain any active ingredients whereby the active ingredient release at a temperature of 24 °C is 1% at most after 20 minutes determined in purified water at 150 rpm using the dissolution test apparatus Ph. Eur./USP equipped with the Basket method and the active ingredient release at a temperature of 37 °C is above 90 % after 45 minutes determined in purified water at 150 rpm using the dissolution test apparatus Ph. Eur./USP equipped with the Basket method.

2. The use of spherical pellets or spherical granules according to claim 1 **characterized in that** the lipid layer formed on the surface of the pellet is of at least 5 µm thickness.

3. The use of spherical pellets or spherical granules according to claim 1 or 2 **characterized in that** the active ingredient is a hydrophilic active ingredient or a hydrophilic salt thereof.

4. The use of spherical pellets or spherical granules according to claim 3 wherein the active ingredient is sodium benzoate.

5. The use of spherical pellets or spherical granules according to any one of the preceding claims **characterized in that** no further coating of said pellets or granules after spheronisation is present.

6. The use of spherical pellets or spherical granules according to any one of the preceding claims **characterized in that** the lipid binder composition comprises
0.1 to 90 wt-% hard fat
0 to 30 wt-% glycerol trimyristate
0 to 30 wt-% glycerol distearate
up to 30 wt-% glycerol dibehenate
whereby either glycerol trimyristate or glycerol distearate are present in an amount of at least 0.1 wt-%.

7. The use of spherical pellets or spherical granules according to any one of the preceding claims wherein the amount of active ingredient in the pellet or granule is in the range of from 0.1 to 80 wt-% of the total pellet or granule.

8. The use of spherical pellets or spherical granules according to any one of the preceding claims **characterized in that** in the step of spheronisation said spheronisation of the extrudate is performed for at least 15 minutes.

9. The use of spherical pellets or spherical granules according to any one of the preceding claims **characterized in that** in the step of spheronisation the temperature of the jacket of the spheroniser is at least 5°C below the melting temperature of the lipid component of the binding composition having the lowest melting temperature.

10. The use of a lipid binder composition for taste-masking comprising at least (i) a hard fat, (ii) either glycerol trimyristate or glycerol distearate, and, (iii) glycerol dibehenate, preferably, said composition comprises
0.1 to 90 wt-% hard fat.
0 to 30 wt-% glycerol trimyristate
0 to 30 wt-% glycerol distearate
up to 30 wt-% glycerol dibehenate
whereby either glycerol trimyristate or glycerol disterate are present in an amount of at least 0.1 wt-%.

## Patentansprüche

1. Verwendung von sphärischen Pellets oder sphärischen Granulaten mit einer Beschichtung aus einer Lipidbindemittelzusammensetzung zur Geschmacksmaskierung erhältlich durch lösungsmittelfreie Kaltextrusion, umfassend die Schritte:
a. Mischen des Wirkstoffs mit der Lipidbindemittelzusammensetzung umfassend mindestens i) ein Hartfett, ii) entweder Glycerintrimyristrat oder Glycerindistearat, und iii) Glycerindibehenat,
b. Kaltextrusion der Mischung,
c. Sphäronisieren des Extrudats, um sphärische Pellets oder sphärische Granulate zu erhalten, **dadurch gekennzeichnet, dass** die Beschichtung auf der Oberfläche der Pellets oder Granulate gebildet wird, die die Pellets oder Granulate vollständig bedeckt, und dass die Beschichtung im wesentlichen keine Wirkstoffe enthält, wobei die Wirkstofffreisetzung bei einer Temperatur von 24°C nach 20 Minuten, bestimmt in gereinigtem Wasser bei 150°C U/min und Verwendung des mit der Korbmethode ausgestatteten Auflösungstestapparates Ph. Eur./USP höchstens 1 % beträgt, und die Wirkstofffreisetzung bei deiner Temperatur von 37°C nach 45 Minuten bestimmt in gereinigtem Wasser bei 150 U/min unter Verwendung der Korbmethode ausgestatteten Auflösungstestgeräts Ph. Eur./USP über 90 % beträgt.

2. Die Verwendung von sphärischen Pellets oder sphärischen Granulaten nach Anspruch 1, **dadurch gekennzeichnet, dass** die Lipidschicht ausgebildet auf der Oberfläche des Pellets mindestens eine Dicke von 5 µm aufweist.

3. Die Verwendung von sphärischen Pellets oder sphärischen Granulaten nach Anspruch 1 oder 2, **dadurch gekennzeichnet, dass** der aktive Inhaltsstoff ein hydrophiler aktiver Inhaltsstoff oder ein hydrophiles Salz davon ist.

4. Die Verwendung von sphärischen Pellets oder sphärischen Granulaten nach Anspruch 3, wobei der aktive Inhaltsstoff Natriumbenzoat ist.

5. Die Verwendung von sphärischen Pellets oder sphärischen Granulaten nach einem der vorherigen Ansprüche, **dadurch gekennzeichnet, dass** keine weitere Beschichtung dieser Pellets oder Granulate nach Sphäronisierung vorliegt.

6. Die Verwendung von sphärischen Pellets oder sphärischen Granulaten nach einem der vorherigen Ansprüche, **dadurch gekennzeichnet, dass** die Lipidbindemittelzusammensetzung umfasst
0,1 bis 90 Gew.-% Hartfett
0 bis 30 Gew.-% Glycerintrimyristat
0 bis 30 Gew.-% Glycerindistearat
bis zu 30 Gew.-% Glycerindibehenat
wobei entweder Glycerintrymyristat oder Glycerindistearat vorhanden sind in einer Menge von mindestens 0,1 Gew.-%.

7. Die Verwendung von sphärischen Pellets oder sphärischen Granulaten nach einem der vorherigen Ansprüche, wobei die Menge am aktiven Inhaltsstoff in dem Pellet oder Granulat von 0,1 bis 80 Gew.-% des Gesamtpellets oder Gesamtgranulats ist.

8. Die Verwendung von sphärischen Pellets oder sphärischen Granulaten nach einem der vorherigen Ansprüche, **dadurch gekennzeichnet, dass** in dem Schritt der Sphäronisierung diese Sphäronisierung des Extrudats mindestens für 15 Minuten durchgeführt wird.

9. Die Verwendung von sphärischen Pellets oder sphärischen Granulaten nach einem der vorherigen Ansprüche, **dadurch gekennzeichnet, dass** bei dem Schritt der Sphäronisierung die Temperatur des Mantels des Sphäronisators mindestens 5°C unterhalb der Schmelztemperatur der Lipidkomponente der Bindemittelzusammensetzung mit der geringsten Schmelztemperatur liegt.

10. Verwendung einer Lipidbindemittelzusammensetzung zur Geschmacksmaskierung umfassend mindestens i) ein Hartfett, ii) entweder Glycerintrimyristrat oder Glycerindistearat, und iii) Glycerindibehenat, bevorzugt umfasst diese Zusammensetzung 0,1 bis 90 Gew.-% Hartfett,
0 bis 30 Gew.-% Glycerintrimyristrat
0 bis 30 Gew.-% Glycerintrimyristat
0 bis 30 Gew.-% Glycerindistearat
bis zu 30 Gew.-% Glycerindibehenat
wobei entweder Glycerintrymyristat oder Glycerindistearat in einer Menge von mindestens 0,1 Gew.-% vorhanden sind.

## Revendications

1. Utilisation de pellets sphériques ou de granulés sphériques ayant un enrobage d'une composition de liant lipidique pour le masquage de goût pouvant être obtenus par extrusion à froid sans solvant comprenant les étapes consistant à
a) mélanger le principe actif avec ladite composition de liant lipidique comprenant au moins (i) une matière grasse dure, (ii) soit du trimyristate de glycérol soit du distéarate de glycérol et (iii) du dibéhénate de glycérol,
b) extruder à froid le mélange,
c) sphéroniser l'extrudat pour obtenir des pellets ou des granulés sphériques,
**caractérisée en ce que** l'enrobage est formé sur la surface desdits pellets ou granulés en recouvrant complètement lesdits pellets ou granulés et **en ce que** l'enrobage ne contient pratiquement pas de quelconques principes actifs, la libération de principe actif à une température de 24 °C déterminée dans de l'eau purifiée à 150 tr/min à l'aide de l'appareil de test de dissolution selon la Ph. Eur./USP pourvu de la méthode à panier étant de 1 % au maximum au bout de 20 minutes et la libération de principe actif à une température de 37 °C déterminée dans de l'eau purifiée à 150 tr/min à l'aide de l'appareil de test de dissolution selon la Ph. Eur./USP pourvu de la méthode à panier étant au-dessus de 90 % au bout de 45 minutes.

2. Utilisation de pellets sphériques ou de granulés sphériques selon la revendication 1 **caractérisée en ce que** la couche lipidique formée sur la surface du pellet a une épaisseur d'au moins 5 µm.

3. Utilisation de pellets sphériques ou de granulés sphériques selon la revendication 1 ou 2 **caractérisée en ce que** le principe actif est un principe actif hydrophile ou un sel hydrophile de celui-ci.

4. Utilisation de pellets sphériques ou de granulés sphériques selon la revendication 3 dans laquelle le principe actif est le benzoate de sodium.

5. Utilisation de pellets sphériques ou de granulés sphériques selon l'une quelconque des revendications précédentes **caractérisée en ce qu'**aucun enrobage supplémentaire desdits pellets ou granulés après sphéronisation n'est présent.

6. Utilisation de pellets sphériques ou de granulés sphériques selon l'une quelconque des revendications précédentes **caractérisée en ce que** la composition de liant lipidique comprend
0,1 à 90 % en poids de matière grasse dure,
0 à 30 % en poids de trimyristate de glycérol,
0 à 30 % en poids de distéarate de glycérol,
jusqu'à 30 % en poids de dibéhénate de glycérol,
soit le trimyristate de glycérol soit le distéarate de glycérol étant présent en une quantité d'au moins 0,1 % en poids.

7. Utilisation de pellets sphériques ou de granulés sphériques selon l'une quelconque des revendications précédentes dans laquelle la quantité de principe actif dans le pellet ou granulé est dans la plage de 0,1 à 80 % en poids du pellet ou granulé total.

8. Utilisation de pellets sphériques ou de granulés sphériques selon l'une quelconque des revendications précédentes **caractérisée en ce que** dans l'étape de sphéronisation, ladite sphéronisation de l'extrudat est effectuée pendant au moins 15 minutes.

9. Utilisation de pellets sphériques ou de granulés sphériques selon l'une quelconque des revendications précédentes **caractérisée en ce que** dans l'étape de sphéronisation, la température de la chemise du sphéroniseur est au moins 5 °C au-dessous de la température de fusion du composant lipidique de la composition liante ayant la plus basse température de fusion.

10. Utilisation d'une composition de liant lipidique pour le masquage de goût comprenant au moins (i) une matière grasse dure, (ii) soit du trimyristate de glycérol soit du distéarate de glycérol et (iii) du dibéhénate de glycérol, de préférence ladite composition comprenant
0,1 à 90 % en poids de matière grasse dure,
0 à 30 % en poids de trimyristate de glycérol,
0 à 30 % en poids de distéarate de glycérol,
jusqu'à 30 % en poids de dibéhénate de glycérol,
soit le trimyristate de glycérol soit le distéarate de glycérol étant présent en une quantité d'au moins 0,1 % en poids.
